Europäisches Patentamt

European Patent Office

Office européen des brevets

(19)

(11) Publication number: **0 369 490**

**A2**

(12) **EUROPEAN PATENT APPLICATION**

(21) Application number: 89121434.8

(51) Int. Cl.⁵: **G01N 33/18**

(22) Date of filing: 20.11.89

(30) Priority: 18.11.88 JP 291900/88
02.03.89 JP 52491/89
31.03.89 JP 82057/89
09.06.89 JP 146999/89
31.03.89 JP 38660/89 U
13.06.89 JP 69379/89 U

(43) Date of publication of application:
23.05.90 Bulletin 90/21

(84) Designated Contracting States:
DE ES FR GB IT

(71) Applicant: **Kao Corporation**
14-10, Nihonbashi Kayabacho 1-chome
Chuo-Ku Tokyo 103(JP)

(72) Inventor: **Sakata, Masaru**
283-24, Hatake, Iwade-cho
Naga-gun Wakayama-ken(JP)
Inventor: **Tanigaki, Masanobu**
845-9, Ootani
Wakayama-shi Wakayama-ken(JP)
Inventor: **Mimura, Koji**
117, Nakaguro, Iwade-cho
Naga-gun Wakayama-ken(JP)
Inventor: **Takaya, Hitoshi**
1130, Nishihama
Wakayama-shi Wakayama-ken(JP)
Inventor: **Konishi, Noriko**
1430, Kada
Wakayama-shi Wakayama-ken(JP)

(74) Representative: **Vossius & Partner**
Siebertstrasse 4 P.O. Box 86 07 67
D-8000 München 86(DE)

(54) **Method of bod measurement and apparatus thereof.**

(57) In a method of measurement of BOD value of test solution by comparing dissolved oxygen decrement $\Delta DO$ of the test solution with dissolved oxygen decrement of standard solution sterilized test solution is used as the standard solution. Apparatus for conducting the above method is also disclosed.

BOD value is calculated using the measured $\Delta DO$ by a relation between 1/BOD and 1/$\Delta DO$.

The apparatus comprises a column for contacting solution with the microorganism, oxygen electrodes disposed before and after the column for measuring the dissolved oxygen value, whereby in an inject condition, the test solution is fed to the oxygen electrode before the column and in the lead condition the test solution is fed to the oxygen electrode after the column, so that $\Delta DO$ can be measured by the two oxygen electrodes.

In the apparatus, the inject condition and the load condition are changed by solenoid valves or rotary valves.

Fig. 2

# METHOD OF BOD MEASUREMENT AND APPARATUS THEREOF

The present invention relates to a method of measurement of BOD (biochemical oxygen demand) value, and more particularly to a method of prompt measurement of BOD value with high accuracy using a BOD sensor made of combination of immobilized microorganism and an oxygen electrode.

BOD means an amount of dissolved oxygen which is necessary to assimilate organic substance in water by aerobic microorganism. BOD is used as a useful basic index of water pollution by organic substance. Way of measurement of BOD is defined in JIS K0102 (Japanese industrial standard K 0102). According to the conventional way of the measurement of BOD, it has taken long time such as five days to measure BOD value with complicated measurement operation and skill. Due to the ground mentioned above, it has been strongly desired to provide a way of measurement of BOD which is able to measure with a short period of time. Recently, there has been developed so called a BOD sensor (Biotechnology and Bioengineering volume XIX pages 1535 to 1547, 1977). The BOD sensor employs such a BOD measurement method that aerobic microorganisms are immobilized on water insoluble carriers and using the immobilized microorganisms and an oxygen electrode, and flowing waste water for contact with the immobilized microorganisms, an amount of oxygen consumption is measured by the oxygen electrode to obtain the BOD value of the drain water.

The BOD measurement using the BOD sensors is such that a relation between the oxygen consumption and BOD is preliminarily obtained.

The BOD measurement is such that a relation between the amount of the oxygen consumption and BOD value is preliminarily obtained using standard solution, then oxygen consumption is measured when waste water is passed , whereby BOD value can be calculated using the relation.

Conventionally, as the standard solution, there has been used gulcose glutamic acid mixed solution (150 mg of glucose and 150 mg of glutamic acid were solved in 1 liter of water) being warmed at a predetermined temperature and diluted.

However, in the conventional method using such standard solution as mentioned above, the accuracy of the measurement is sometimes deteriorated depending on the kind of waste water to be measured.

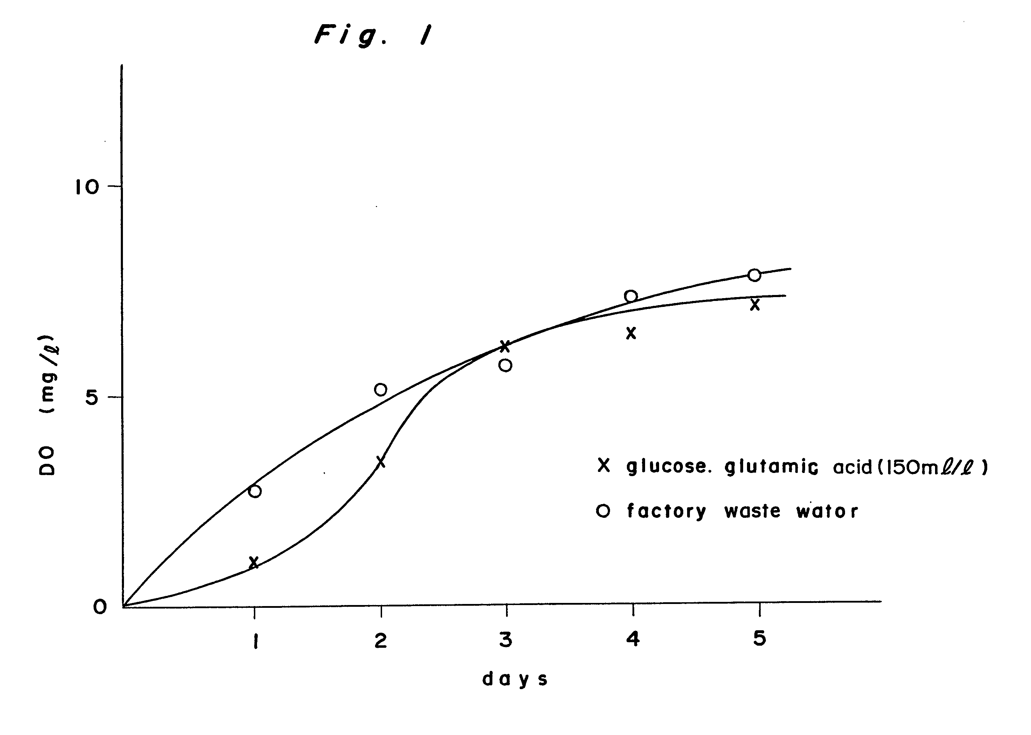

The present inventors have studied the above problem and discovered that the deterioration of the measurement accuracy occurs because the component of the standard solution mentioned above is greatly different from the component of the waste water of factory, whereby all kinds of drain water do not show similar consumption activity(or BOD curve). In particular, at an initial step of the biological assimilation of organic substances, as shown in Fig. 1, the standard solution shows quite different activity from that of the real waste water. In case the BOD measurement is made in a short period of time by using the BOD sensor, if the BOD value of the waste water is calculated from the BOD analytic curve using glucose, glutamic acid standard solution, a large error occurs in the value of the measurement. Moreover, in case where bacteria in activated sludge for processing the waste water to be measured are used as the microorganism to be immobilized on the insoluble carriers, since the active sludge bacteria have been acclimated with the waste water to be measured, the error becomes large when a standard solution which is different from the waste water to be measured.

## SUMMARY OF THE INVENTION

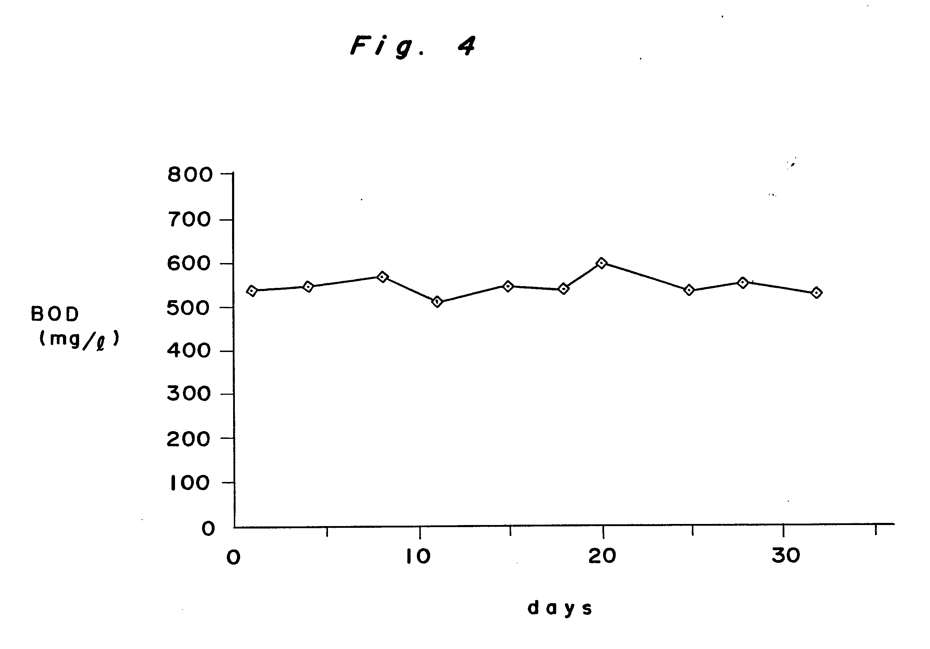

The present inventors have further studied to solve the problem mentioned above, they have discovered that such solution that is taken from the waste water of a factory or a waste water treatment facilities to be measured and sterilized shows a BOD curve similar to the BOD curve of the waste water to be measured and it is possible to measure BOD value correctly if the sterilized solution of the waste water is used as the standard solution. Moreover, as shown in Fig. 4, the sterilized solution of the waste water may be preserved for a long time of about more than one month at a room temperature if the solution is preserved in a sealed condition.

An essential object of the present invention is to provide a method of measuring BOD value which is able to measure the BOD value rapidly with a high accuracy.

Another object of the present invention is to provide a device for measuring BOD value suitable for conducting the method of the measurement of BOD value according to the present invention.

In order to accomplish the object of the present invention, according to the present invention, there is provided a method of measuring BOD value using a BOD sensor which measures BOD value by contacting waste water to be measured with immobilized aerobic microorganism and then by detecting decreased oxygen value, whereby BOD value is obtained by comparing the measured decreased oxygen value of the waste water to be measured and a decreased oxygen value of standard solution, wherein the sterilized solution of waste water to be measured is used as the standard solution.

In the present invention, it is an essential feature to use the sterilized solution of waste water to be measured as the standard solution.

The use of such sterilized waste water as the standard solution makes it possible to measure the BOD value quickly with a high accuracy since the activity of oxygen consumption in the standard solution is generally equal to that of the waste water to be measured.

According to the prior art, it has never been expected to use the sterilized waste water as the standard solution, because it has been considered that since there are mixed a lot of floating solid substances in the waste water, whereby there may occur occlusion of such solid substances in a filter provided in the BOD sensor, on the other hand, in case such floating solid substances are preliminarily filtered out, there can be obtained different BOD value.

However, according to the study by the inventors, it is discovered that the BOD value of the waste water obtained by filtering out such a size of floating solid substances that do not cause occlusion inside the column packed with the immobilized microorganism is substantially same as the BOD value of the original waste water to be measured. By this knowledge, it become possible to use the waste water to be measured as the standard solution in the BOD sensor, and as a result of further study, the present invention is completed.

## BRIEF EXPLANATION OF THE DRAWINGS

Fig. 1 shows graphs of the oxygen decrement value with respect to lapse of days,

Fig. 2 is a schematic diagram showing an apparatus for performing the method of BOD measurement according to the present invention,

Fig. 3 is another embodiment of an apparatus for performing the method of BOD measurement according to the present invention,

Figs. 4 and 5 are graphs showing BOD values with respect to the days,

Fig. 6 is a scehmatic diagram showing an arrangement for sterilizing the solution by an ultraviolet lamp,

Fig. 7 is a block diagram of a control device used in the apparatus for measuring BOD value according to the present invention,

Fig. 8 is a flow chart showing the operation of the control device,

Figs. 9 and 10 show graphs of the relation between BOD and ΔDO,

Fig. 11 is a schematic diagram showing another embodiment of the apparatus for measuring BOD value according to the present invention,

Figs. 12 and 13 are graphs of the relation between BOD and ΔDO,

Figs. 14 to 19 are schematic diagrams showing a further embodiment of the BOD measurement apparatus according to the present invention,

Figs. 20 and 21 are graphs showing relations between DO values and time,

Figs. 22 and 23 are schematic diagrams showing a still further embodiment of the BOD measuring apparatus according to the present invention,

Figs. 24 and 25 are graphs showing relations between DO values and time,

Fig. 26 is a schematic diagram showing a conventional BOD measurement device,

Figs. 27 and 28 are graphs showing relations between DO values and time measured by the apparatus shown in Fig. 26.

Figs. 29 to 34 are schematic diagrams showing various embodiments of the BOD measurement apparatus according to the present invention,

Fig. 35 is a graph showing a relation between the pressure at the inlet port of the column and time,

Figs. 36 and 37 are graphs showing relations between DO value and time.

## DETAILED DESCRIPTION OF THE PREFERRED EMBODIMENTS

In the present invention, as the sterilization method, there may be used autoclaving method, filter sterilization method. However, in the filter sterilization method, the BOD component may be undesirably excluded, therefore, it may be possible to use the drain water as the standard solution of the BOD sensor in case the concentration of organic substances contained in the waste water is low. However it is difficult to

EP 0 369 490 A2

use the waste water as the standard in case the concentration of organic substances contained in waste water is high.

Accordingly, preferably autoclaving method is used. The conditions for the sterilization are preferably 110° to 130°C and 15 to 30 minutes. However, higher temperature and longer time period than the above may be employed without any problem.

The BOD value of the sterilized waste water is measured according to the method provided by any standard defined in the respective countries, for example, JIS K0102 in Japan. The sterilized waste water is used as the standard solution. The standard solution may be preserved in container in effective condition for a long time so long as the container is sealed. Therefore it is possible to provide a lot of sterilized waste water and to use a desired volume of the sterilized waste water when BOD measurement is conducted. If the drain water contains relatively large floating solid substances, they should be filtered out. As previously mentioned, such filtering does not affect the accuracy of the measured BOD value.

The size of the mesh of the filter is preferably 100 to 300 μm, such filter mesh size depending on the kind of the floating substances contained in drain water.

In carrying out the present invention, any known BOD sensor may be used in addition to various BOD sensors mentioned later. As the BOD sensor, there may be used a non-separate type composed of immobilized microorganism and oxygen electrode arranged in one body or alternatively column type in which the immobilized microorganism and the oxygen electrode are separated.

As the aerobic substances, there is no limitation but it is preferable to use microorganism in an activated sludge tank which the waste water to be measured is treated.

One advantage of the present invention is in that the BOD measurement can be conducted easily and accurately by using the sterilized drain water as the standard solution.

Another advantage of the present invention is in that measurement of the BOD value can be automatically conducted during a long period since the standard solution preserved in a tank is available by sampling during the period of measurement. Therefore, the method of the present invention is preferably used to measure automatically the BOD value for a long time with a high accuracy in such a circumstance that the organic substance concentration of waste water to be measured is kept constant within a certain range.

EXAMPLE 1

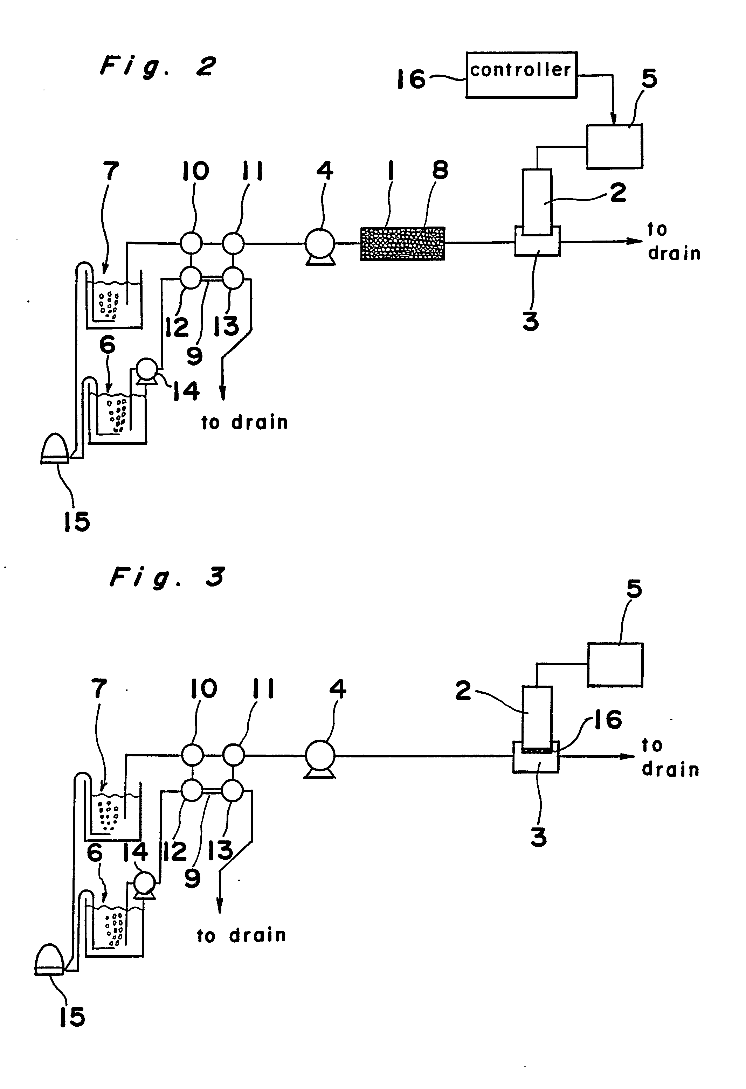

BOD value was measured using a column type BOD sensor as shown in Fig. 2 comprising an oxygen electrode and a container containing immobilized microorganism.

Predetermined volume of sample solution to be measured was filled in a sample loop 9, thereafter the sample solution was transferred to a container 1 in which immobilized microorganism 8 was contained by operation of valves 10 to 13, whereby the amount of the oxygen consumption was measured and the BOD value was calculated. The capacity of the sample loop 9 was 0.2 mℓ and the flow rate of buffer solution was 2.5 mℓ/min.. As the microorganism, microorganism contained in an activated sludge tank which the waste water to be measured was treated was being progressed was used with immobilizing process.

As the standard solution there was used such solution that a possible amount of the waste water to be measured was taken and sterilized for 20 minutes at 120°C in an autoclave and the BOD value was measured in accordance with JIS K0102. It is noted that the sample solution and the waste water to be measured were respectively filtered with a filter of mesh diameter 150 μm.

The result of measurement is shown in table 1. As apparent from the table 1, the BOD value measured by the method of the present invention well coincided with the measurement value measured by JIS K0102.

4

TABLE 1

| SAMPLE NUMBER | BOD VALUE BY JIS | BOD VALUE BY BOD SENSOR |
|---|---|---|
| | (mg/ℓ) | (mg/ℓ) |
| 1 | 37 | 33 |
| 2 | 52 | 50 |
| 3 | 81 | 84 |
| 4 | 130 | 126 |
| 5 | 180 | 180 |
| 6 | 255 | 253 |

COMPARISON 1

BOD values were measured in the same manner as shown in the example 1 except that the standard solution was glucose glutamic acid mixed solution. The result is shown in the table 2. In this case the BOD values measured by the BOD sensor do not coincide with those measured by JIS method.

TABLE 2

| SAMPLE NUMBER | BOD VALUE BY JIS | BOD VALUE BY BOD SENSOR |
|---|---|---|
| | (mg/ℓ) | (mg/ℓ) |
| 1 | 37 | 93 |
| 2 | 52 | 150 |
| 3 | 81 | 286 |
| 4 | 130 | 526 |
| 5 | 180 | 1059 |
| 6 | 255 | 3256 |

EXAMPLE 2

BOD values were measured using a direct contact type BOD sensor as shown in Fig. 3 which comprises immobilized microorganism 16 and the oxygen electrode 2.

Predetermined volume of sample solution to be measured was filled in a sample loop 9, thereafter the sample solution was transferred to a cell 3 in which immobilized microorganism 16 was contained by operation of valves 10 to 13, whereby the amount of the oxygen consumption was measured and the BOD value was calculated. The capacity of the sample loop 9 was 1 mℓ and the flow rate of buffer solution was 3 mℓ/min.. As the microorganism, microorganism contained in an activated sludge tank which the waste water to be measured was treated was used with immobilizing process.

As the standard solution there was used such solution (570, 285, 114 mg/ℓ) that a possible amount of the waste water to be measured was taken and sterilized for 20 minutes at 120 °C in an autoclave and the BOD value was measured in accordance with JIS K0102. It is noted that the sample solution and the waste water to be measured were respectively filtered with a filter of mesh diameter 150 μm.

The result of measurement is shown in table 3. As apparent from the table 3, the BOD value measured by the method of the present invention well coincided with the measurement value measured by JIS K0102.

TABLE 3

| SAMPLE NUMBER | BOD VALUE BY JIS | BOD VALUE BY BOD SENSOR |
|---|---|---|
| | (mg/ℓ) | (mg/ℓ) |
| 7 | 80 | 75 |
| 8 | 109 | 108 |
| 9 | 169 | 173 |
| 10 | 200 | 202 |
| 11 | 225 | 233 |
| 12 | 319 | 324 |

COMPARISON 2

BOD values were measured in the same manner as shown in the example 2 except that the standard solution was glucose glutamic acid mixed solution. The result is shown in the table 4. In this case the BOD values measured by the BOD sensor do not coincide with those measured by JIS method.

TABLE 4

| SAMPLE NUMBER | BOD VALUE BY JIS | BOD VALUE BY BOD SENSOR |
|---|---|---|
| | (mg/ℓ) | (mg/ℓ) |
| 7 | 80 | 110 |
| 8 | 109 | 172 |
| 9 | 169 | 323 |
| 10 | 200 | 411 |
| 11 | 225 | 526 |
| 12 | 319 | 1059 |

EXAMPLE 3

BOD values were measured in the same manner as those in the example 1 except that there was used such standard solution which the waste water to be measured was sterilized by ultraviolet rays of 254 nm wavelength, 200 mw/cm$^{-2}$ for one hour.

The result of measurement is shown in table 5. As apparent from the table 5, the BOD value measured by the method of the present invention well coincided with the measurement value measured by JIS K0102.

TABLE 5

| SUMPLE NUMBER | BOD VALUE BY JIS | BOD VALUE BY BOD SENSOR |
|---|---|---|
| | (mg/ℓ) | (mg/ℓ) |
| 1 | 37 | 31 |
| 2 | 52 | 52 |
| 3 | 81 | 90 |
| 4 | 130 | 133 |
| 5 | 180 | 185 |
| 6 | 255 | 251 |

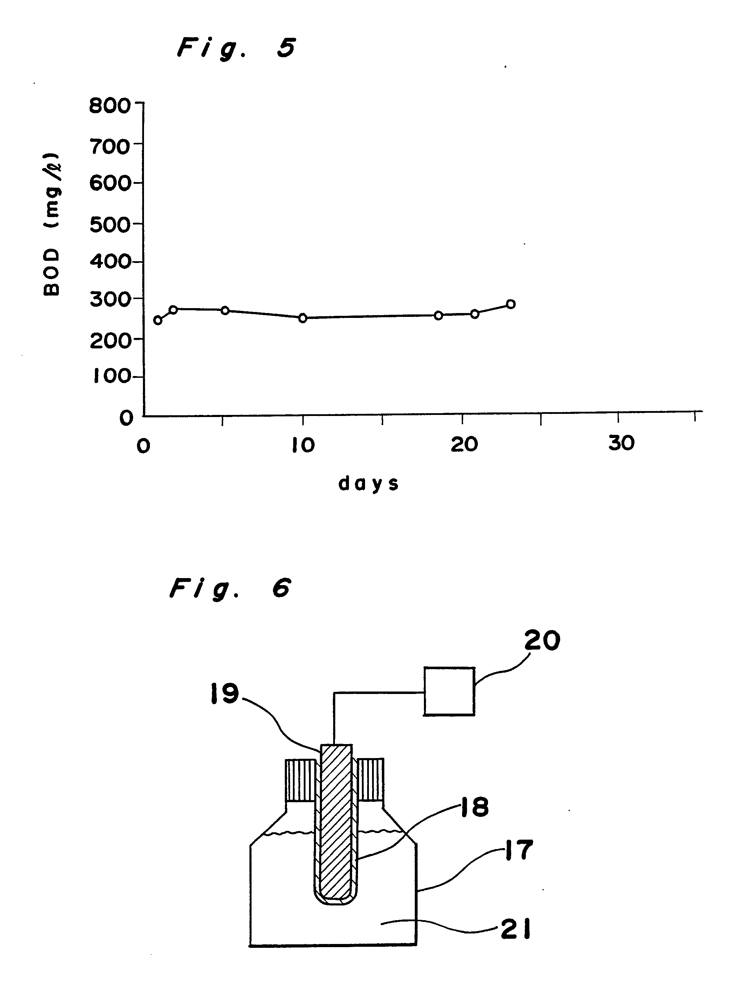

The ultra violet ray sterilization was conducted using such a device as shown in Fig. 6 in which an ultra violet lamp 19 is inserted in a heat resist container 17 standing vertically in the container 17 through a quartz protection tube 18. The waste water 21 is filled in the container 17 and the ultraviolet lamp 19 is illuminated by a power source 20 so that the waste water 21 is sterilized.

A method of measuring and calculating the BOD value and device therefor will be hereinafter described in detail.

There is provided a control device 16 for controlling the valves 10 to 13 and calculating the BOD value using the result of measurement by the recorder 5.

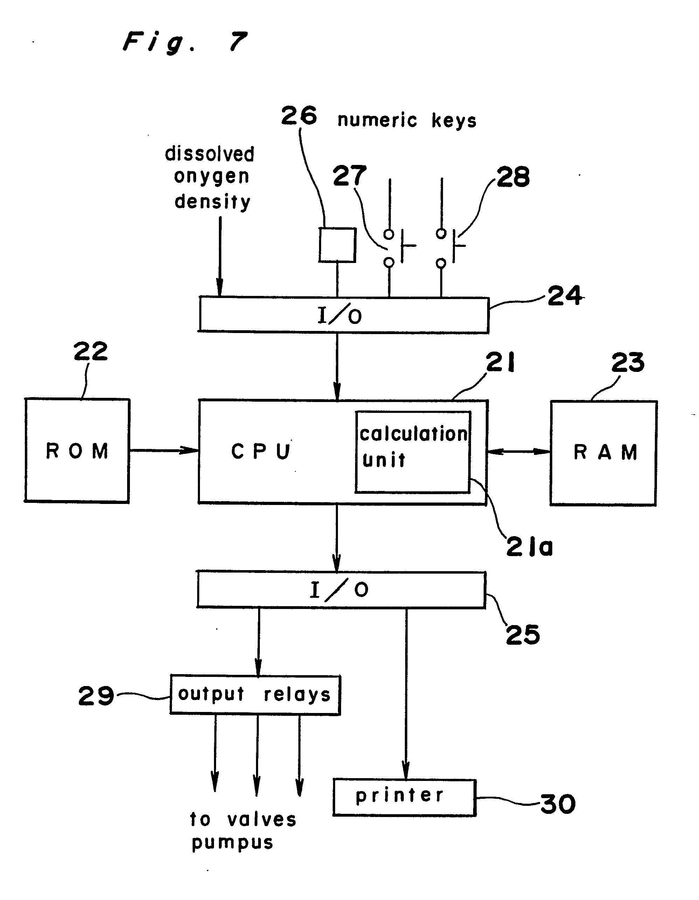

Referring to Fig. 7, a central processing unit (CPU)21 is provided with a calculation unit 21a for calculating constants A and B by least square method in a equation

$$BOD = \frac{B \cdot \Delta DO}{A - \Delta DO} \qquad \ldots \ldots (1)$$

which calculates ΔDO (expressed as calibration) representing the amount of decrement of oxygen from the standard solution of which BOD value is already known.

A ROM 22 (read only memory) stores control programs used in CPU 21.

A RAM 23 stores various numerical values such as BOD value and equation (1) and so on in addition to various data to perform control and calculation as the BOD sensor.

Various devices are connected to CPU 21 through I/O interfaces 24 and 25. 26 denotes numeric keys 0-9 for inputting BOD values and necessary data, 27 a calibration switch for conducting the calibration, 28 a measurement switch for conducting the measurement for obtaining BOD value of the sample to be measured. 29 output relays for operating the valves and pumps by the instruction from CPU 21. 30 denotes a printer for printing result of measurement of the BOD value of the sample waste water to be measured.

The signal of the dissolved oxygen electrode indicating the concentration of the oxygen in the flow cell 3 is inputted in the I/O 24.

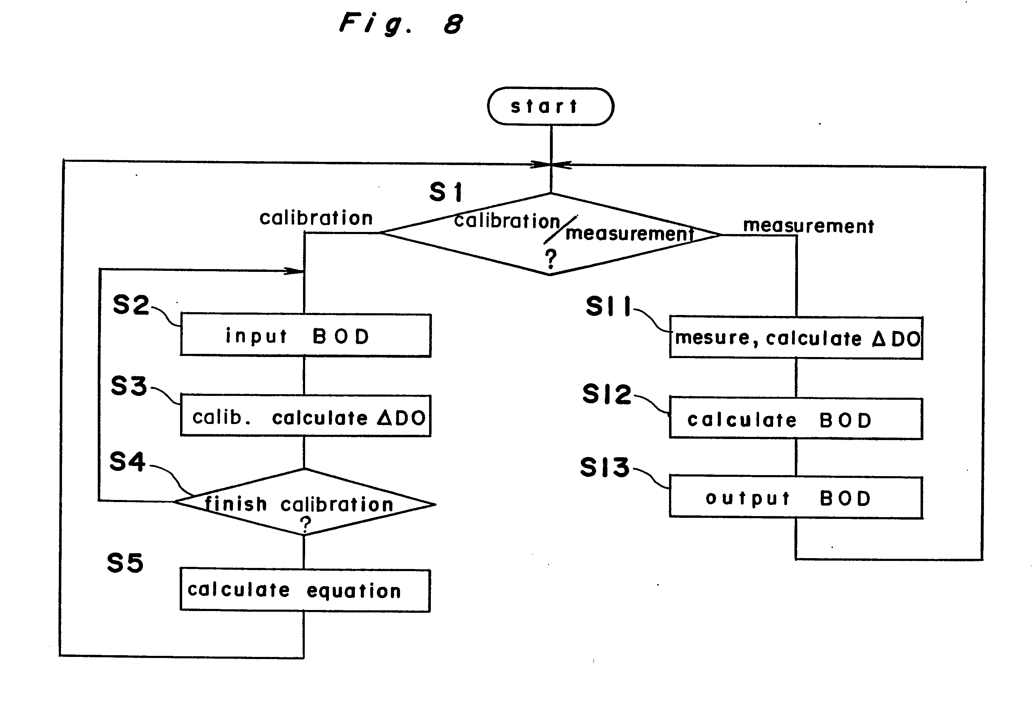

An operation of the control device shown in Fig. 7 is explained with reference to a flow chart in Fig. 8.

In the step S1, it is judged that which of calibration or measurement. In case of calibration, the standard solution of which BOD value is known is filled in the container 6, thereafter the calibration switch 27 is operated.

As the standard solution, the sterilized waste water to be measured is used.

By operation of the calibration switch 27, the program goes to the step S2 for waiting for input of BOD value of the standard solution. When the BOD value is input by numerical keys 26, the calibration is conducted in the step S3 in such a manner that the valves 10 to 13 are changed from such a condition that the buffer solution is transported to the container 1 through the valves 10 and 11 and the standard solution is discharged from a drain port through the valves 12 and 13 to another condition that the buffer solution which substantially does not contain the organic substance passes a path of valves 10 → 12 → 13 → 11 and the standard solution in the path 9 is transported to the container 1.

The organic substrates in the standard solution are assimilated, whereby oxygen is consumed in the container 1 and the dissolved oxygen concentration is measured by the oxygen electrode 2 in the flow cell 3 and the oxygen meter 5, then from the oxygen concentration measured, the oxygen decrement value ΔDO is calculated in the CPU 21 of the control device 15.

It is detected in the step S4 whether or not the calibration mentioned above is completed.

In order to determine the equation (1) using BOD value and the measured $\Delta DO$, it is necessary to calibrate on the two kinds of the standard solution of which BOD value are different at least.

Since the constants A and B in the equation (1) are obtained by least square method, the greater the number of time of the calibration, the higher accuracy of the equations can be made.

The operation of the least square method in the calculation unit 21 is described.

It is assumed that the following basic equation is established,

[1]

$$\frac{1}{\Delta DO} = \frac{K}{DOm} \cdot \frac{1}{BOD} + \frac{1}{DOm} \qquad \cdots\cdots (2)$$

wherein DOm and K/DOm are constants.

where; $x = 1/BOD$, $y = 1/\Delta DO$,

$$Y = \frac{K}{DOm} x + \frac{1}{DOm}$$

By modifying the above equation,

$$x = \frac{DOm \cdot y - 1}{K}$$

$$BOD = \frac{K}{DOm/\Delta DO - 1}$$

$$= \frac{K \cdot \Delta DO}{DOm - \Delta DO}$$

$$= \frac{(K/DOm)\Delta DO}{1 - (1/DOm)\Delta DO}$$

By replacing 1/DOm and K/DOm by constants a and b

$$BOD = \frac{b \cdot \Delta DO}{1 - a\Delta DO} = \frac{b/a \cdot \Delta DO}{1/a - \Delta DO}$$

By replacing 1/a and 1/b by A and B, the following equation

$$BOD = \frac{B \cdot \Delta DO}{A - \Delta DO} \qquad \cdots\cdots (1)$$

can be obtained. This is called as Lineweaver-Burk Plot.

[2] By making the following equation as a basic equation,

$$\frac{BOD}{\Delta DO} = \frac{K}{DOm} + \frac{1}{DOm} \cdot BOD$$

where; $x = BOD$, $y = BOD/\Delta DO$,

$$y = \frac{K}{DOm} + \frac{1}{DOm} x$$

modifying the above equation $x = DOm \cdot y - K$

$$BOD = DOm \frac{BOD}{\Delta DO} - K$$

$$= \frac{K}{DOm/\Delta DO - 1}$$

$$= \frac{(K/DOm)\Delta DO}{1-(1/DOm)\Delta DO}$$

in a similar manner as the modification of the case [1],

$$BOD = \frac{B \cdot \Delta DO}{A - \Delta DO} \qquad \cdots \cdots (1)$$

can be obtained. This is called as Hotstee Plot method.

[ 3 ]

$$\Delta DO = DOm - K \frac{\Delta DO}{BOD}$$

where; $x = \Delta DO/BOD$, $y = \Delta DO$,
$y = DOm - Kx$
can be obtained.
Replacing DOm and K as 1/b and a/b

$$y = \frac{1}{b}(1-ax)$$

$$\frac{y}{x} = \frac{1}{b}(\frac{1}{x} - a)$$

$$BOD = DOm(\frac{BOD}{\Delta DO} - \frac{K}{DOm})$$

$$= \frac{K}{DOm/\Delta DO - 1}$$

$$= \frac{(K/DOm)\Delta DO}{1-(1/DOm)\Delta DO}$$

Similar to the modification in the case [1],

$$BOD = \frac{B \cdot \Delta DO}{A - \Delta DO} \qquad \cdots \cdots (1)$$

can be obtained. This is called as Eadie Plot method.
In general, the calculation [1] needs a heavy load when BOD is measured on the solution by which BOD value is low.

The calculation [2] needs a heavy load when BOD is measured on the solution of which BOD value is high.

The calculation [3] is sensitive against the error of the straight line.

Accordingly, any one of the calculations [1] to (3) should by manually or automatically selected depending on the BOD ΔDO curve so that an accurate BOD measurement can be made.

When a predetermined number of times of ΔDO calibration of the standard solution is completed, the program goes to the step S5 in which the constants A and B in the equation (1) using the inputted BOD and measured ΔDO in the calculation unit 21a and the obtained equation (1) is stored in RAM 23. The program goes to the step S1.

Subsequently, in order to perform the BOD measurement, the waste water to be measured is filled in the container 6 and the measurement switch 8 is operated.

By the above operation, the program goes to the step S11 from S1 in which ΔDO is measured in the same manner as performed in obtaining ΔDO of the standard solution. In the subsequent step S12, BOD value of the drain water to be measured is calculated by the equation (1) using the ΔDO and the result of the calculation is outputted in the step S13 for example by a printer.

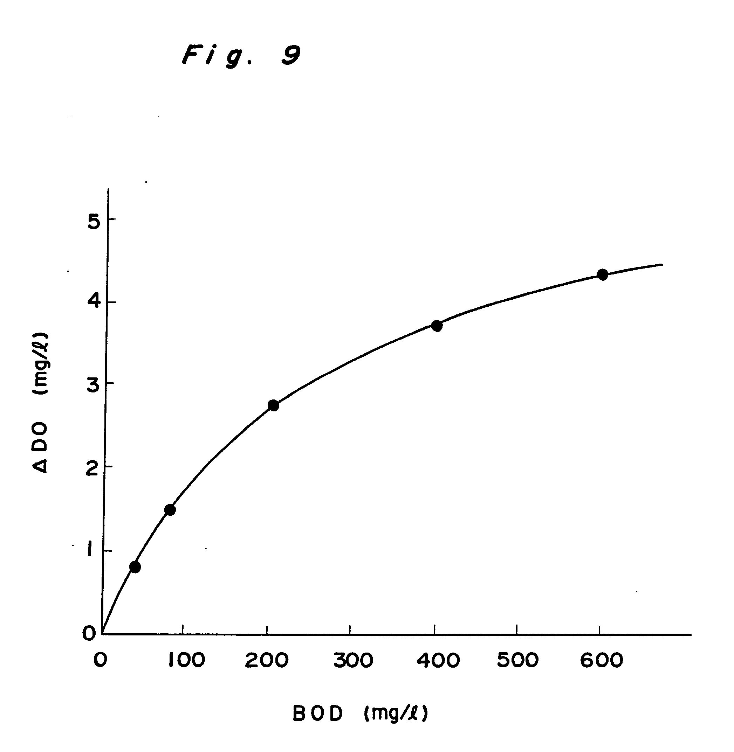

Fig. 9 is a graph showing a relation between the BOD value in the horizontal axis and ΔDO in the vertical axis. The BOD values of the respective standard solutions were 40 mg/ℓ, 80 mg/ℓ, 200 mg/ℓ, 400 mg/ℓ and 600 mg/ℓ.

As shown in Fig. 9, in a range smaller than 100 mg/ℓ of the BOD values, the relation is substantially linear, but in a range larger than 100 mg/ℓ of the BOD value, the relation is non linear.

When the constants A and B of the equation (1) are calculated by least square method using the respective BOD values of the standard solutions and their ΔDO, A = 6.31 and B = 260.59 are obtained by the calculation in the calculation unit 21a.

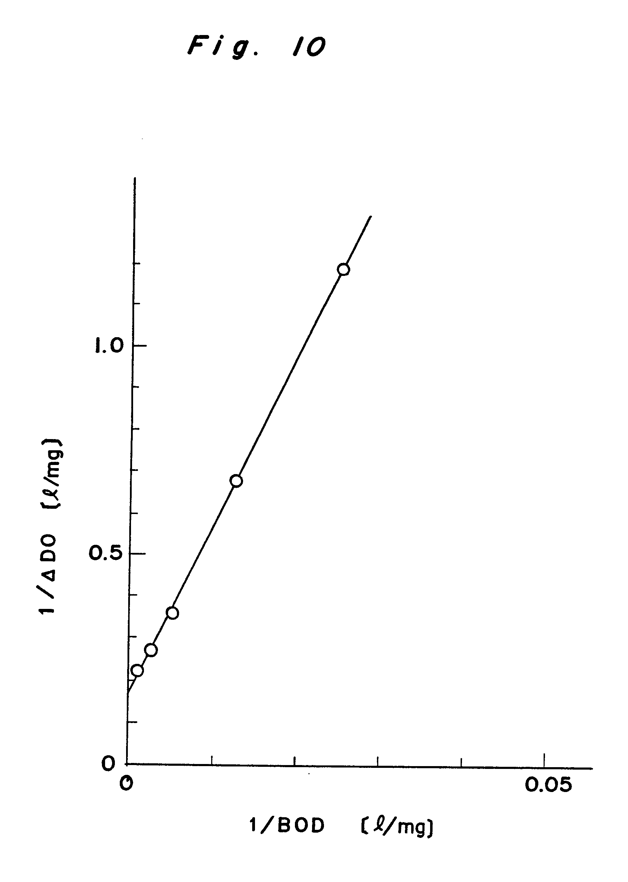

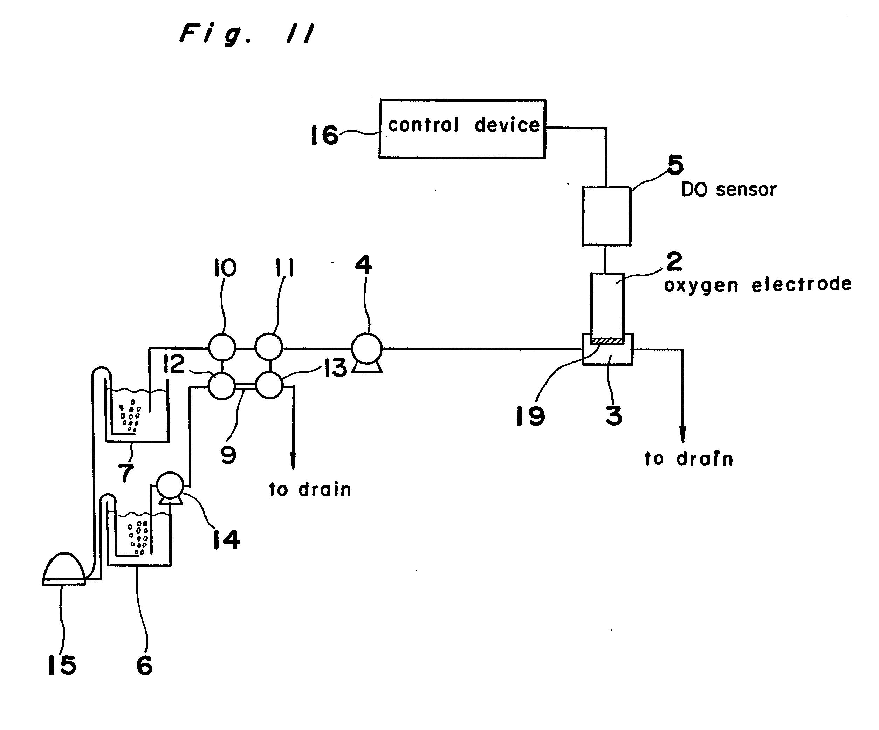

A straight line L in Fig. 10 shows the relation of the equation (1) obtained by the calculation in the calculation unit 21a with the horizontal axis taken for 1/BOD and the vertical axis taken 1/ΔDO. The circles in the graph show respective ΔDO values corresponding to the BOD values of the respective standard solutions.

As understood from the graph in Fig. 10, the measured ΔDO values are coincided with the straight line L. Accordingly, by inputting the measured ΔDO in a equation representing the relation of the straight line L, accurate BOD value can be obtained.

Table 6 shows results of the measurement of BOD values using the measuring device shown in Figs. 1 and 7.

TABLE 6

| SAMPLE NUMBER | BOD VALUE BY JIS | BOD VALUE BY BOD SENSOR |
|:---:|:---:|:---:|
| | (mg/ℓ) | (mg/ℓ) |
| 1 | 18 | 21 |
| 2 | 63 | 66 |
| 3 | 112 | 110 |
| 4 | 230 | 236 |
| 5 | 275 | 271 |
| 6 | 338 | 330 |
| 7 | 451 | 449 |
| 8 | 532 | 529 |

From the table 6, it can be seen that accurate BOD values can be measured for wide range of BOD value.

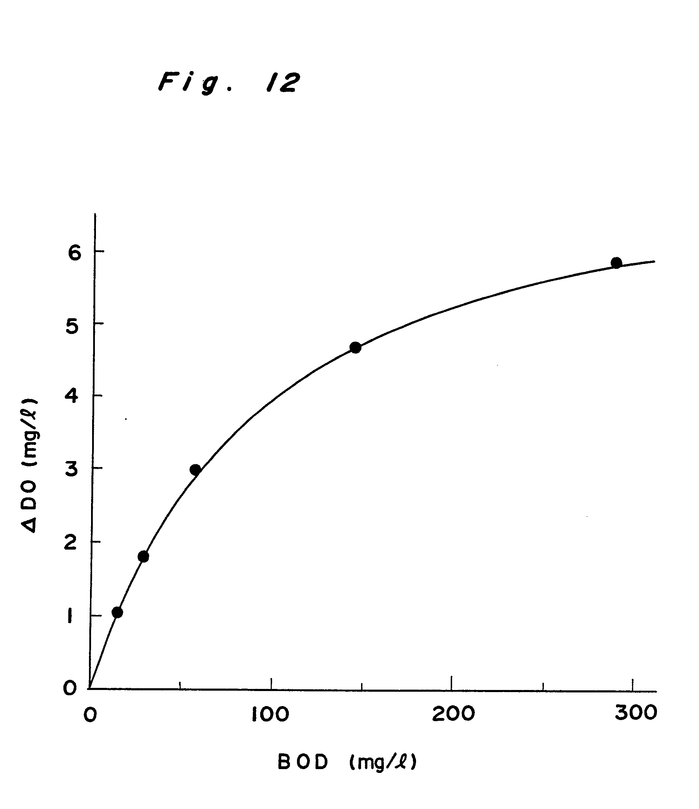

Fig. 11 shows a further example 4 of the BOD measurement device using so called a direct contact type BOD sensor in which the output port of the pump 4 is coupled directly to the flow cell 3 without the container 1 and the oxygen electrode 2 is provided at its bottom surface a immobilized microorganism layer.

In the example the capacity of the loop 9 was 1ml, the amount of flow of the buffer solution was 3ml/min.

Oxygen decrement values ΔDO of the standard solution of 15 mg/ℓ, 30 mg/ℓ, 60 mg/ℓ, 145 mg/ℓ and 290 mg/ℓ of BOD value were measured using the device shown in Fig. 11 and the results are plotted as shown in Fig. 12.

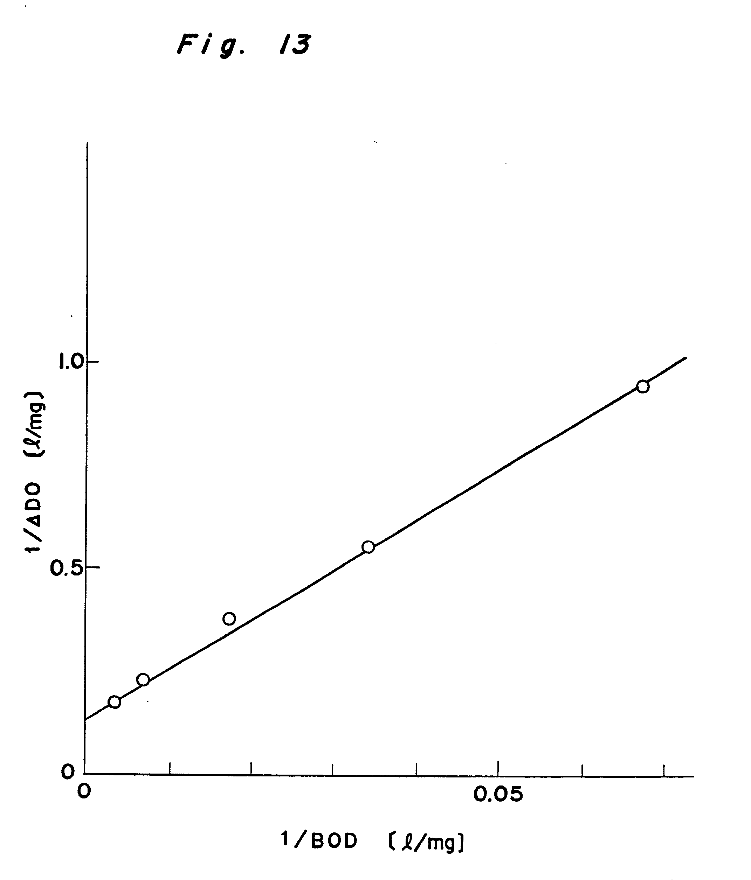

In the example using the arrangement shown in Fig. 11, there were calculated by the calculation unit 21a the constants A = 7.72 and B = 94.03 in the equation (1) and 1/BOD and 1/ΔDO were shown in Fig. 13.

Table 7 shows the result of the measurement in the example using the device shown in Fig. 11.

TABLE 7

| SAMPLE NUMBER | BOD VALUE BY JIS | BOD VALUE BY BOD SENSOR |
|---|---|---|
| | (mg/ℓ) | (mg/ℓ) |
| 9 | 10 | 12 |
| 10 | 38 | 41 |
| 11 | 67 | 65 |
| 12 | 98 | 102 |
| 13 | 148 | 142 |
| 14 | 175 | 168 |
| 15 | 230 | 229 |
| 16 | 298 | 305 |

From the table 7, it is understood that accurate BOD value can be obtained over a wide range of BOD value.

Although in the above examples, only a predetermined limited amount of the sample solution in the loop 9 is used, there may be used such a arrangement that the sample solution is continuously supplied to the container 1 or flow cell 3.

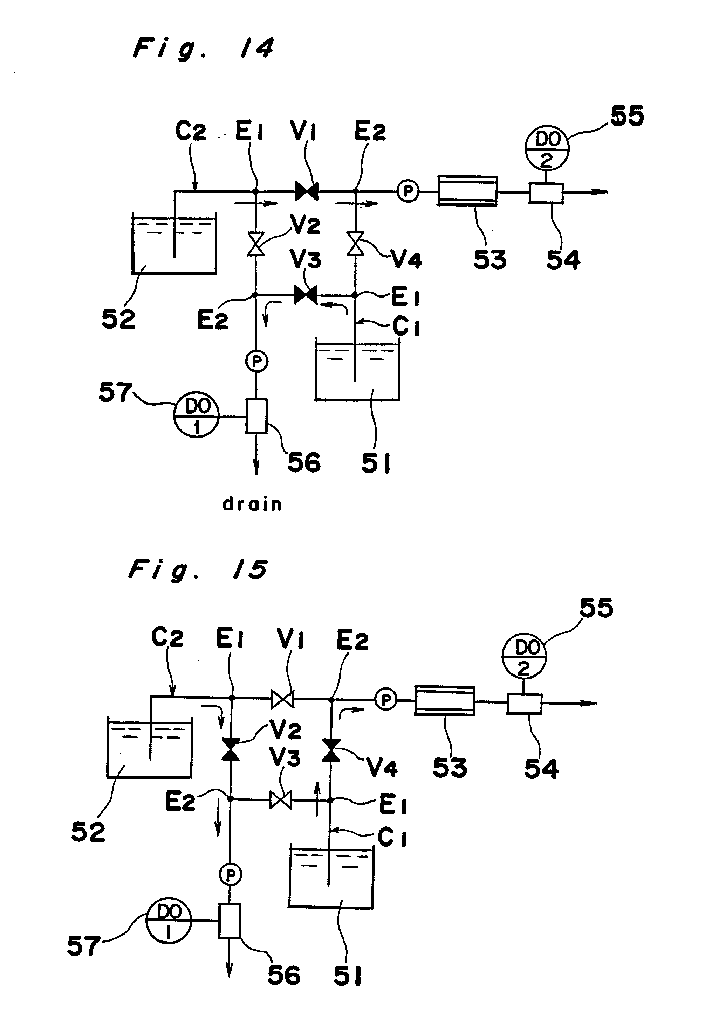

A further embodiment of a BOD measuring device according to the present invention is shown in Figs. 14 and 15.

51 denotes test solution which the BOD value is to be measured, 52 buffer solution and in each solution 51 and 52 oxygen value is in saturated condition by blowing air into each solution 1 and 2 by an air pump (not shown). V1 to V4 are switching valves coupled in a bridge type, of which common junction points E1 are coupled with a connecting pipe C1 and C2 which receive the test solution 51 and the buffer solution 52 respectively. 53 denotes a column in which microorganism is immobilized, 54 a flow cell provided at output port of the column, 55 a DO electrode for measuring the dissolved oxygen in the respective test solution and the buffer solution.

One of the common joints E2 is coupled to an inlet port of the column 53 and another joint E2 is coupled to the flow cell 56 and DO electrode 57. A character P in Fig. 15 denotes pumps for feeding solution disposed in pipes.

The operation of the BOD measurement device shown in Figs. 14 and 15 is explained hereinafter.

After the valves V1 and V3 are opened (shown in dark mark) with the valves V2 and V4 closed, the test solution 51 and the buffer solution 52 are fed to the joints E1 respectively by means of pumps.

The test solution is fed to the drain through the valve V3 and flow cell 56, whereby the dissolved oxygen concentration $DO_{1s}$ of the test solution is measured by the DO electrode 57. The buffer solution 52 is fed to the flow cell 54 through the valve V1 and column 53 and the dissolved oxygen concentration $DO_{2b}$ of the buffer solution 52 of which oxygen is consumed by the breathing of the microorganism in the column 53.

The condition shown in Fig. 14 in which the buffer solution 52 is fed continuously to the column 53 is referred to as a load condition. The load condition is continued for about 10 minutes, thereafter the condition changes to an inject condition shown in Fig. 15.

In Fig. 15, the valves V2 and V4 are opened with the valves V1 and V3 closed. In this condition, since the test solution 51 is fed to the column 53 through the valve V4, the dissolved oxygen $DO_{2s}$ is measured by the DO electrode 55 in the flow cell 54. The detected dissolved oxygen $DO_{2s}$ is the dissolved oxygen concentration of the test solution 51 in which the oxygen is consumed by the breathing of the microorganism and assimilation of the organic substrate. On the other hand, the buffer solution 52 is fed to the flow cell 56 through the valve V2 and the dissolved oxygen concentration $DO_{1b}$ is measured by the DO electrode 57.

The oxygen decrement value $\Delta DO$ consumed by the assimilation of the organic substance in the test solution 51 is expressed

$$\Delta DO = (DO_{1s}-DO_{2s})-(DO_{1b}-DO_{2b})$$

In case where the oxygen decrement value $\Delta DO$ is obtained, the BOD value of the test solution can be obtained by the known manner.

In the measurement device mentioned above, since any flow cell is not disposed at the inlet port side of the column 53 as used in the prior art, it is possible to eliminate such a disadvantage that the buffer solution 52 and the test solution 51 are mixed at the time of changing from the load condition to the injection condition, whereby it becomes possible to measure the dissolved oxygen concentration in short after the change to the inject condition.

This advantage can occurs when the condition changes from the inject condition into the load condition.

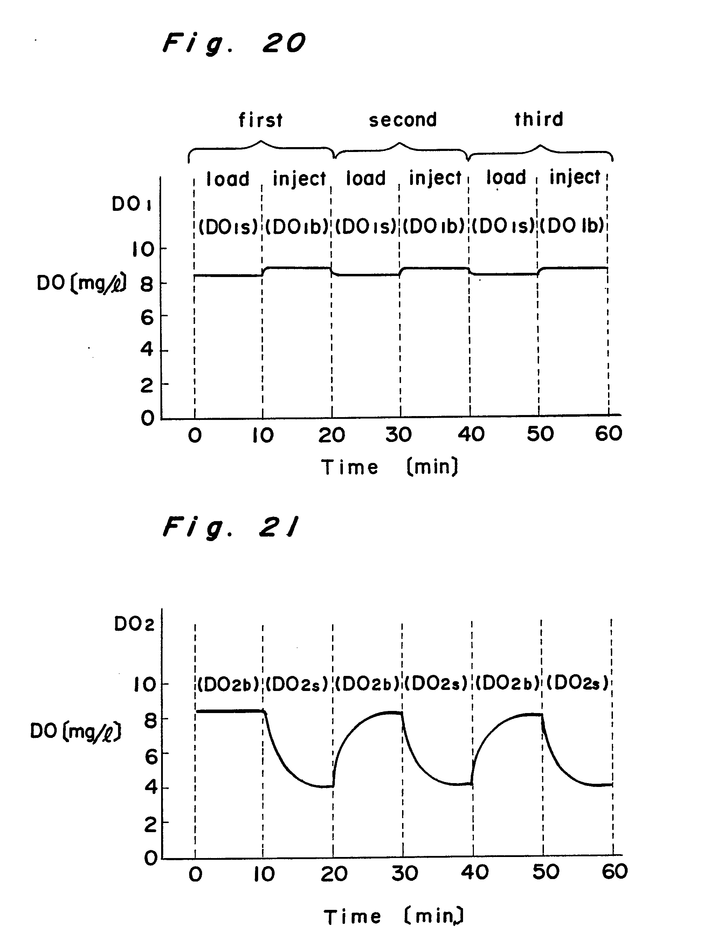

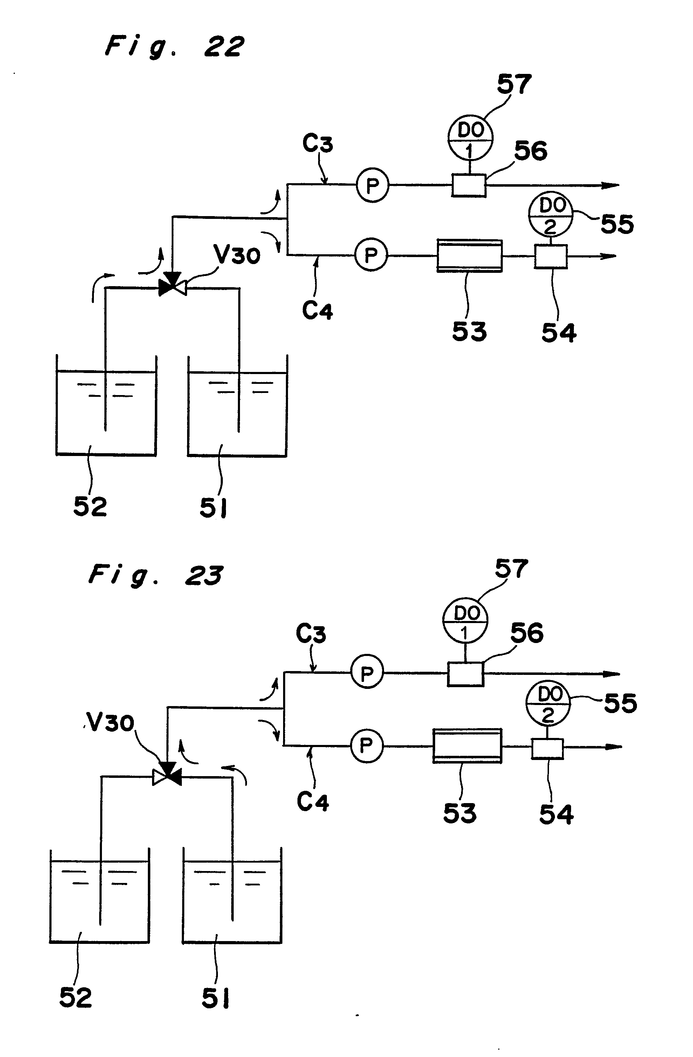

Fig. 20 shows the dissolved oxygen concentration $DO_1(DO_{1b}, DO_{1s})$ measured in the flow cell 54 and Fig. 22 shows the dissolved oxygen density $DO_2(DO_{2b}, DO_{2s})$ measured in the flow cell 56.

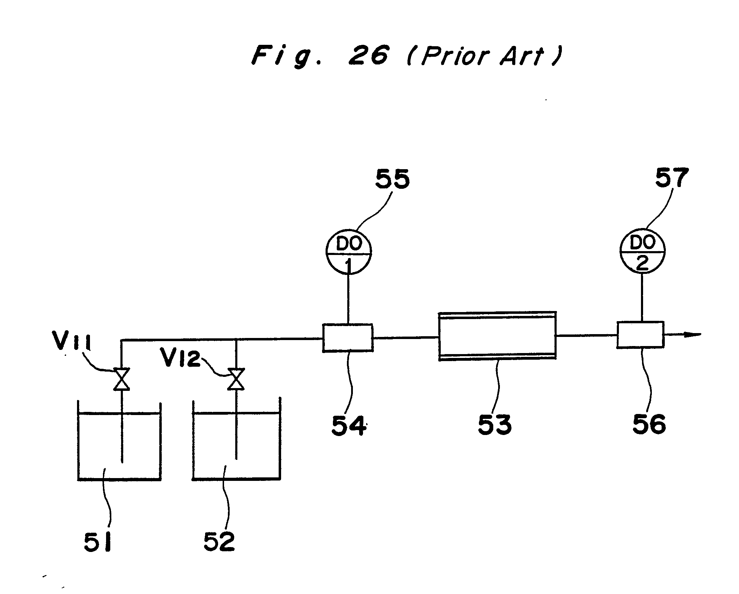

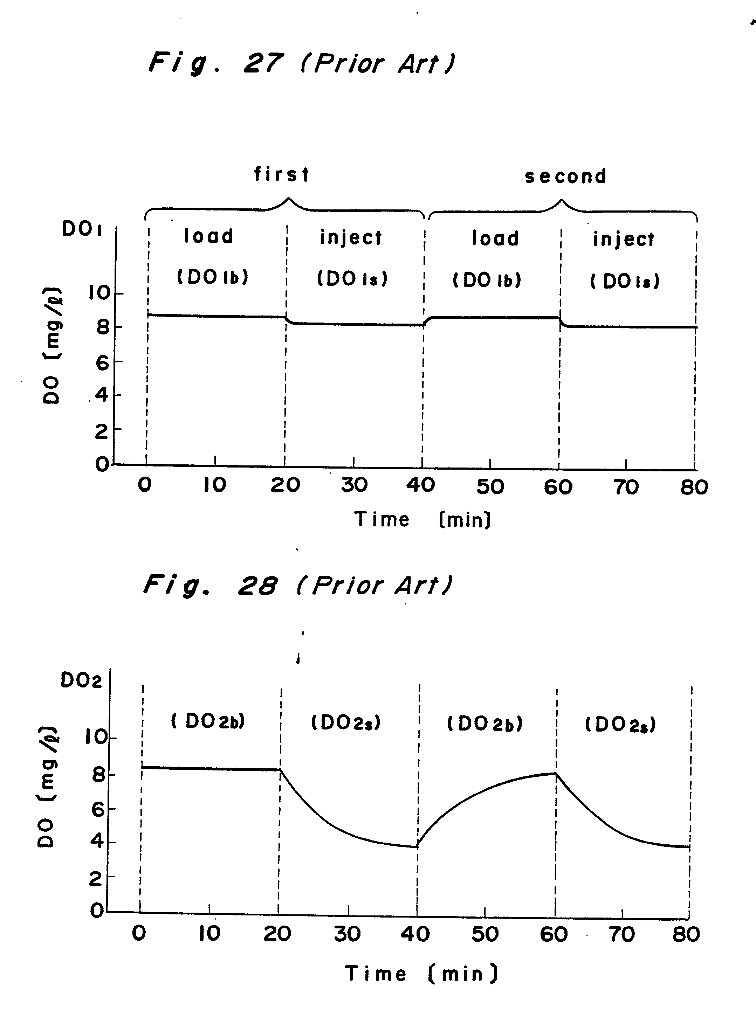

As understood by comparison of Fig. 21 and Fig. 28 showing the DO measurement in the prior art device shown in Fig. 26, the time during which the dissolved oxygen concentration is stabilized after the load condition is changed to the injection condition is short, therefore in the device shown in Figs. 14 and 15 it becomes possible to measure the BOD value in short period after such change of the solution feeding condition.

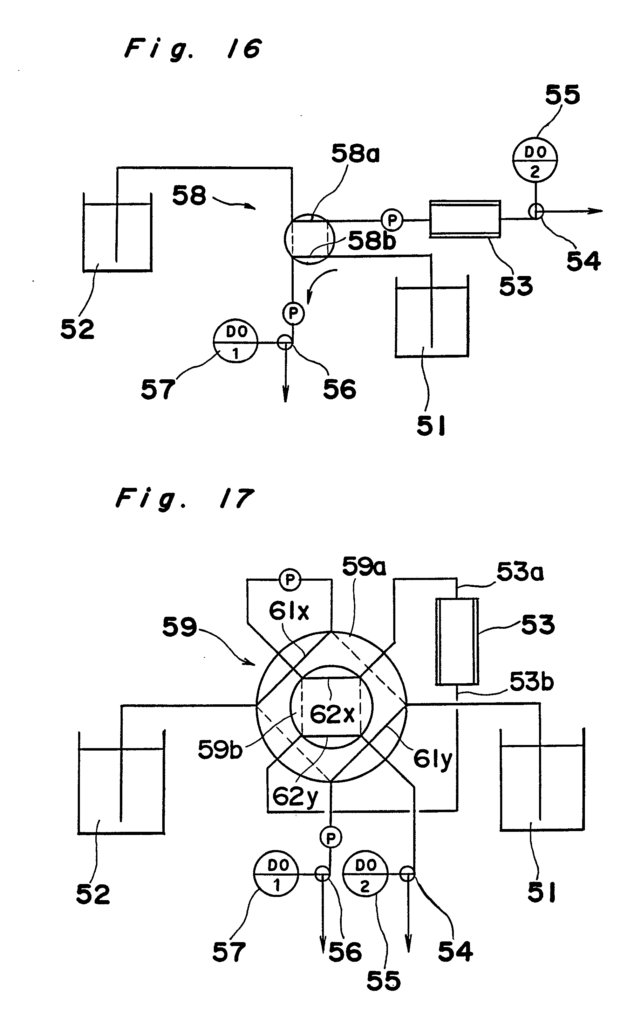

A further embodiment of the BOD measurement device according to the present invention, in the embodiment shown in Fig. 16, there is used a rotary valve 58 in place of the valves V1 to V4. In the load condition, the rotary valve 58 is set in such a manner that solution paths 58a and 58b are positioned as shown in solid lines so that the test solution 51 is fed to the flow cell 56 and the buffer solution 52 is fed to the column 53. In the inject condition, the rotary valve 58 is rotated by a predetermined angle and the solution paths 58a and 58b are positioned as shown in dotted lines so that the test solution 51 and the buffer solution 52 flow to the column 53 and the flow cell 54 through the paths shown in the dotted lines.

In the embodiment shown in Fig. 15, in a case where large insoluble substances are contained in the test solution, in the inject condition, there may occur deposition of such insoluble substances in the column 53, thereby causing jam of the substances to be occurred and to feed the solution to the column 53 may be prevented.

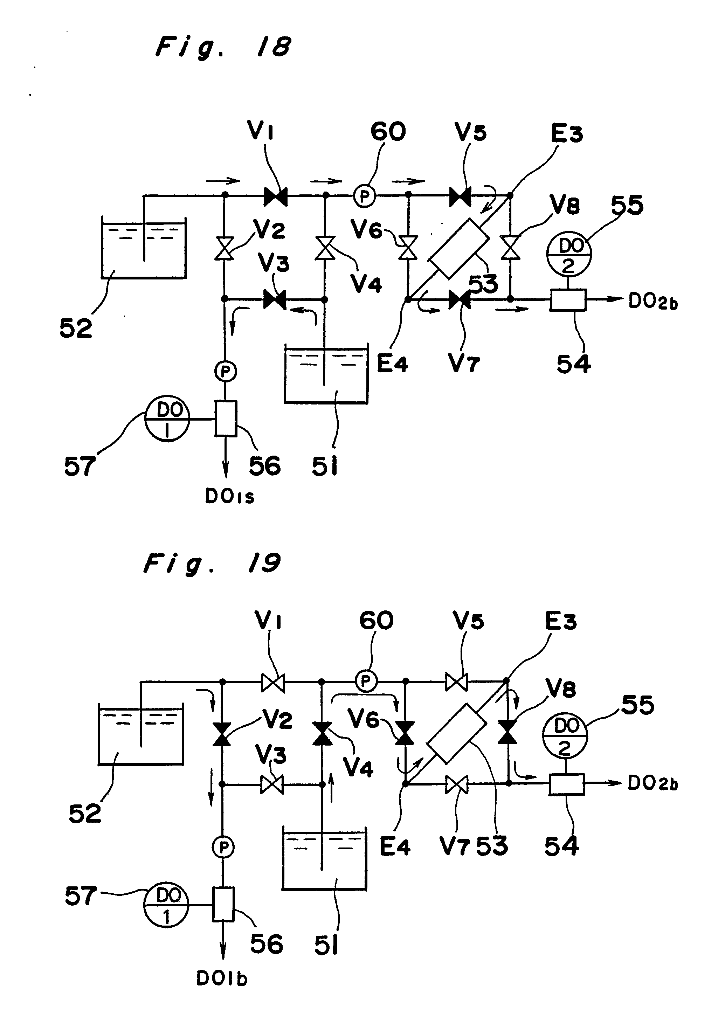

In order to solve the problem mentioned above, a further embodiment of the BOD measurement device according to the present invention as shown in Figs. 18 and 19, in which the blowing direction of the solution can be reversed in the load condition and inject condition.

Referring to Figs. 18 and 19, there are provided additional four valves V5 to V8 between the pump 60 feeding the solution from the valves V1 or V4 and the flow cell 54. The column 53 is coupled between the common junction E3 of the valves V5 and V8 another common junction E4 of the valves V6 and V7. In the arrangement, in the loading condition, as shown in Fig. 18, the valves V5 and V7 are opened with the valves V6 and V8 closed so that the buffer solution 52 flows the column 53 in a direction from E3 to E4. In the inject condition, the valves V6 and V8 are opened with the valves V5 and V7 closed so that the test solution 51 flows the column 53 in a direction from E4 to E3. In this arrangement, the insoluble substances deposited in the column 53 during inject condition can be discharged as drain by the buffer solution flowing in the reversed direction, whereby jam of the insoluble substances on the column 53 can be prevented.

Fig. 17 shows a further embodiment of the BOD measurement device according to the present invention wherein the valves V1 to V8 are replaced by a rotary valves 59 having two rotary members 59a and 59b. The rotary member 59a has changeable paths 61x and 61y and the rotary member 59b has changeable paths 62x and 62y.

In the load condition the buffer solution 52 passes the rotary valve 59 through the paths shown in the solid lines and flows in the column 53 through the port 53a, on the other hand in the inject condition the test solution 51 passes the rotary valve 59 through the paths shown in the dotted lines and flows in the column 53 through the port 53b so that flow of the direction of the solution in the column 53 can be reversed, whereby jam of the insoluble substances in the column 53 can be prevented.

Figs. 22 and 23 show a further embodiment of the BOD measurement device according to the present invention. There is provided a three way valve V30 is provided so as to feed the test solution 51 or buffer solution 52 to the pipes C3 for drain and pipe C4 for column 53. In the drain pipe C3 there is provided the flow cell 56 and the oxygen electrode 57. In the column pipe C4 the flow cell 54 and the oxygen electrode 56 are provided.

In operation, in the load condition as shown in Fig. 22 only the buffer solution 52 is fed to the drain pipe C3 and column pipe C4 through the valve V30. The dissolved oxygen concentration $DO_{1b}$ of the buffer solution 52 flowing the drain pipe C3 can be measured by the oxygen electrode 57 in the flow cell 56. In the column pipe C4, the dissolved oxygen concentration $DO_{2b}$ of the buffer solution 52 in which oxygen is consumed by the breathing of the microorganism in the column 53 can be measured by the oxygen electrode 55 in the flow cell 54.

When ten minutes elapses in the load condition, the condition is changed to the inject condition.

As shown in Fig. 23, only the test solution 51 is fed to the drain pipe C3 and column pipe C4 by switching the valve V30. The dissolved oxygen concentration $DO_{1s}$ of the test solution 51 flowing the pipe C3 is measured by the oxygen electrode 57 in the flow cell 56. The dissolved oxygen concentration $DO_{2s}$ of the test solution in which oxygen is consumed by the breathing of the microorganism in the column 3 and assimilation of the organic substance included in the test solution 51 can be measured. By the measurement values there can be obtained the oxygen decrement value $\Delta DO$ of the test solution 51 by the assimilation of the organic substance of the test solution 51 as follows:

$\Delta DO = (DO_{1s}-DO_{2s})-(DO_{1b}-DO_{2b})$.

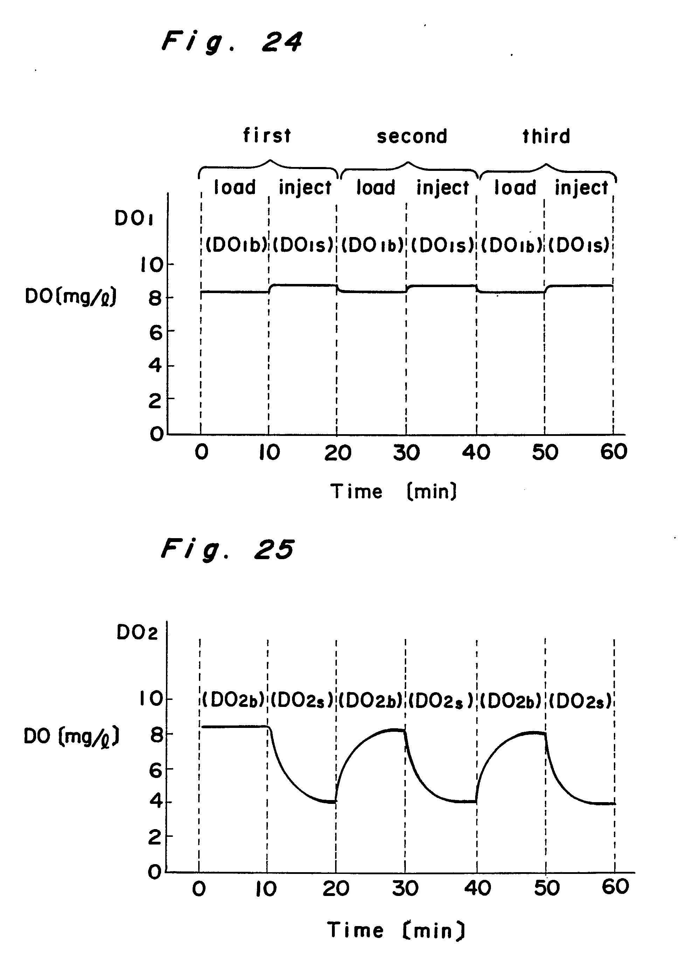

Fig. 24 shows the dissolved oxygen concentration $DO_1(DO_{1b}, DO_{1s})$ measured in the flow cell 55 and Fig. 25 shows the dissolved oxygen concentration $DO_2(DO_{2b}, DO_{2s})$ measured in the flow cell 54, it can be understood by Figs. 24 and 25 that the period of time during which the dissolved oxygen concentration is stabilized from the switching period becomes short.

In the above embodiment, in place of the three way valve V30 there may be used two two way valves. As the operation for measuring $\Delta DO$ used in this embodiment, there may be applied the embodiments shown in Figs. 14 and 15 and Figs. 18 and 19, namely in the load condition the valves V3 and V4 are closed with the valves V1 and V2 opened, on the other hand in the inject condition the valves V1 and V2 are closed with the valves V3 and V4 opened.

In the BOD measurement device shown in Figs. 14 to 19 since the oxygen concentration of the solution before passing the column is measured in the drain pipe, it is possible to eliminate a flow cell in the path before the column, therefore, mixture of the buffer solution and test solution do not flow for a long time, whereby an accurate and quick measurement can be made.

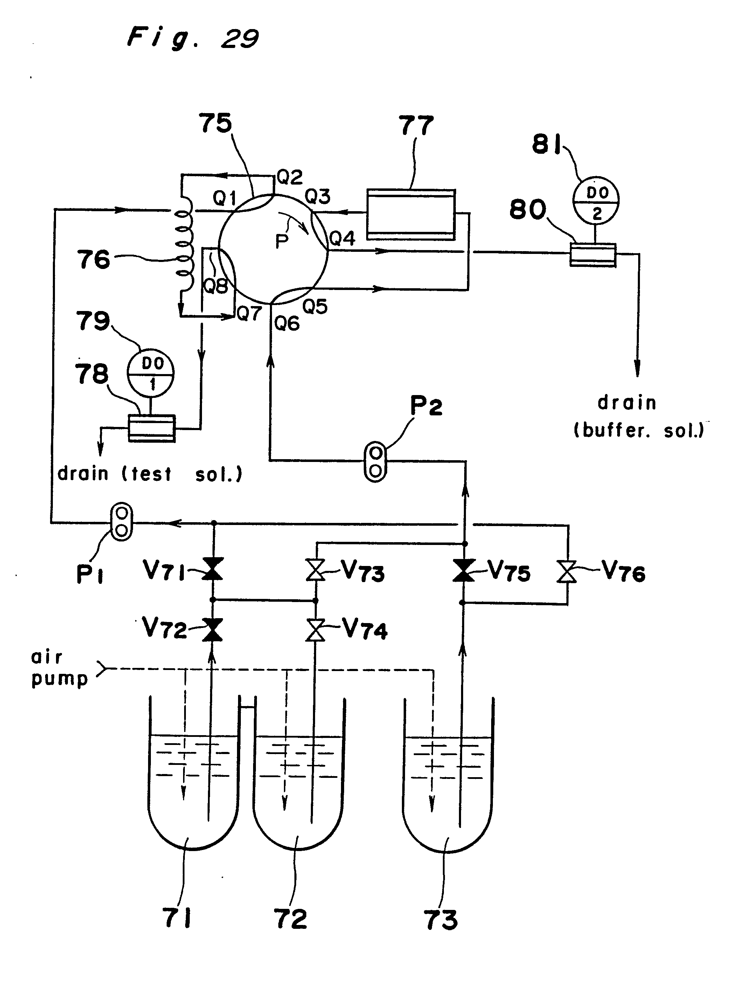

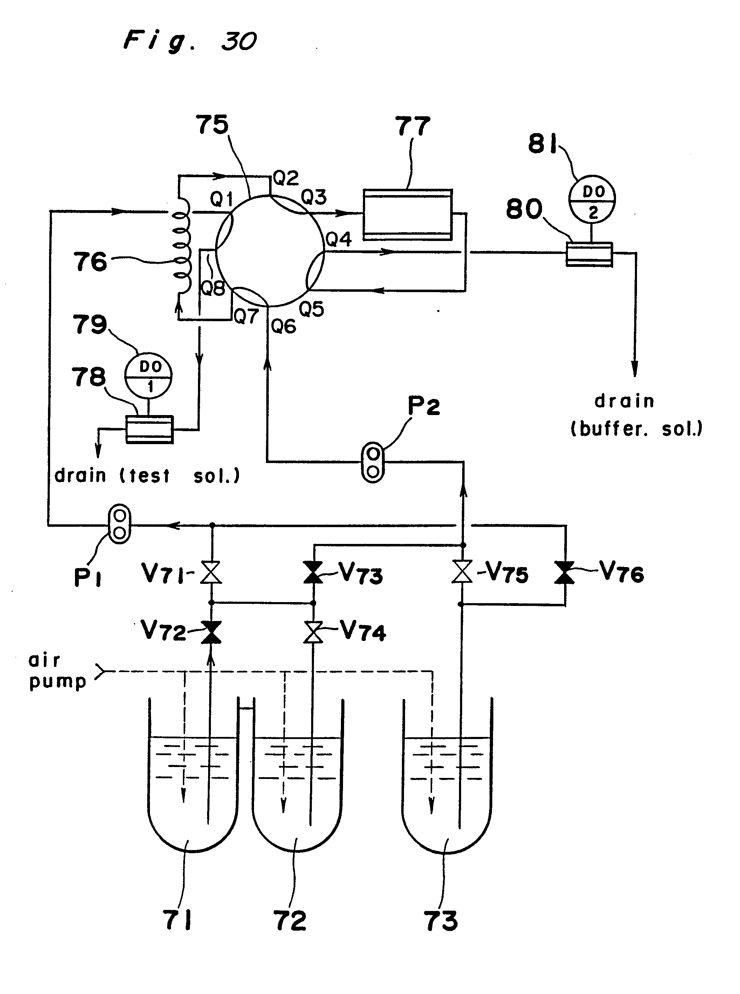

A still further embodiment of the BOD measurement device according to the present invention is shown in Figs. 29 and 30, in which 71 denotes test solution to be measured, 72 standard solution of known BOD value, 73 buffer solution. The respective solutions 71, 72, 73 are saturated by oxygen which is fed by an air pump (not shown).

V71 to V76 are valves and P1 and P2 are pumps. By operating the valves V71 to V76 and pumps P1 and P2 selectively, the solutions 71 to 73 can be selectively fed to the desired as mentioned later.

In the load condition shown in Fig. 29 pipes Q1 and Q2, Q3 and Q4, Q5 and Q6 and Q7 and Q8 are connected respectively by paths of a rotary valve 75.

In the inject condition shown in Fig. 30, by rotating the rotary valve 75 by a predetermined angle, pipes Q2 and Q3, Q4 and Q5, Q6 and Q7 and Q8 and Q1 are respectively connected by the rotary pump 75.

The outlet port of the pump P1 is connected with the pipe Q1 and the outlet port of the pump P2 is connected with the pipe Q6.

76 denotes a sample loop for storing a predetermined volume of the test solution 71 or standard solution 72. The sample loop 76 is disposed between the pipes Q7 and Q2.

A cylindrical column 77 for storing the immobilized microorganism is connected between the pipes Q3 and Q5. A flow cell 78 is connected with the drain pipe Q8. 79 denotes a DO electrode for measuring the dissolved oxygen concentration in each solution in the flow cell 78.

81 denotes a DO electrode for measuring the dissolved oxygen concentration in the flow cell 80.

There are two kinds of method for measuring BOD values. One method is so called a steady state method in which the buffer solution and the test solution are alternatively fed to the column 77 continuously for a predetermined period. In this method the sample loop 76 acts merely a pipe for feeding the solution. Another method is so called a flow injection method in which a predetermined volume of the test solution is stored in the sample loop 76 and the stored test solution of the predetermined volume is fed to the column 77 by pushing the test solution by the buffer solution . This operation is repeated over a plurality of times.

The flow injection method is suitable for measuring the BOD value of the test solution having a high BOD value.

An operation of the device shown in Figs. 29 and 30 under the steady state method is explained.

The rotary valve 75 is set in the condition as shown in Fig. 29. The valves V71, V72 and V75 are opened with the valves V73, V74 and V76 are closed and the pumps P1 and P2 are driven.

The test solution 71 is fed from the pipe Q1 to the pipe Q2 through the rotary valve 75 by the pump P1. Moreover, the test solution passes to the pipes Q7 and Q8 through the sample loop 76, reaching the flow cell 78, thereafter being discharged as a drain.

13

On the other hand, the buffer solution 73 passes the valve V75 and the ports Q6 and Q5 in turn passes the column 77 from the right end in the drawing, further passes Q3 and Q4, coming in the flow cell 80, being discharged as the drain.

This load condition is continued for 10 minutes. During this 10 minutes the dissolved oxygen concentration $Do_{1s}$ of the test solution is measured by the DO electrode 79, while the dissolved oxygen concentration $DO_{2b}$ of the buffer solution after passing the column 77 is measured by the DO electrode 81.

After the loading operation is completed, the rotary valve 75 is rotated by a predetermined angle in a direction shown by an arrow mark R so as to set the rotary valve 75 as shown in Fig. 30, and the valves V72, V73 and V76 are opened with the valves V71, V74 and V75 closed. Thus the test solution 71 is fed to the left port of the column 77 by the pump $P_2$ through the valves V72, V73, Q6, Q7, sample loop 76, Q2 and Q3 in the above mentioned order. After passing the column 77, the test solution passes the ports Q5 and Q4 and the flow cell 80, being discharged.

On the other hand, the buffer solution 73 passes the valve V76, the ports Q1 and Q8 and the flow cell 78 by the pump P2 and is discharged.

The inject condition is kept for 10 minutes.

During this inject condition, the dissolved oxygen concentration $DO_{1b}$ of the buffer solution is measured by the DO electrode 79 and the dissolved oxygen concentration $DO_{2s}$ of the test solution after the test solution is passed the column 77 is measured by the DO electrode 81.

When the inject condition is completed, the next load condition is started. In this manner the load condition and inject condition are alternately repeated every ten minutes.

By using the following equation

$$\Delta DO = \Delta DO_s - \Delta DO_b$$
$$= (DO_{1s} - DO_{2s}) - (DO_{1b} - DO_{2b})$$

the oxygen decrement $\Delta DO$ of the test solution can be obtained.

The required BOD value of the test solution may be obtained using the $\Delta DO$ mentioned above by various known calculation method or the calculation method described in the embodiments of the present invention, for example, as shown in Figs. 7, 8, 9 and 10 and the equation (1).

In the inject condition, the insoluble substances contained in the test solution may be deposited in the column 77 and jam of the insoluble substances may be Occurred. However, in the arrangement shown in Figs. 29 and 30, since the buffer solution is flown in the column 77 in the counter direction in the load condition against the flow of the solution in the inject condition, the deposited substances can be discharged as drain, whereby such jam of the insoluble substances in the column can be prevented.

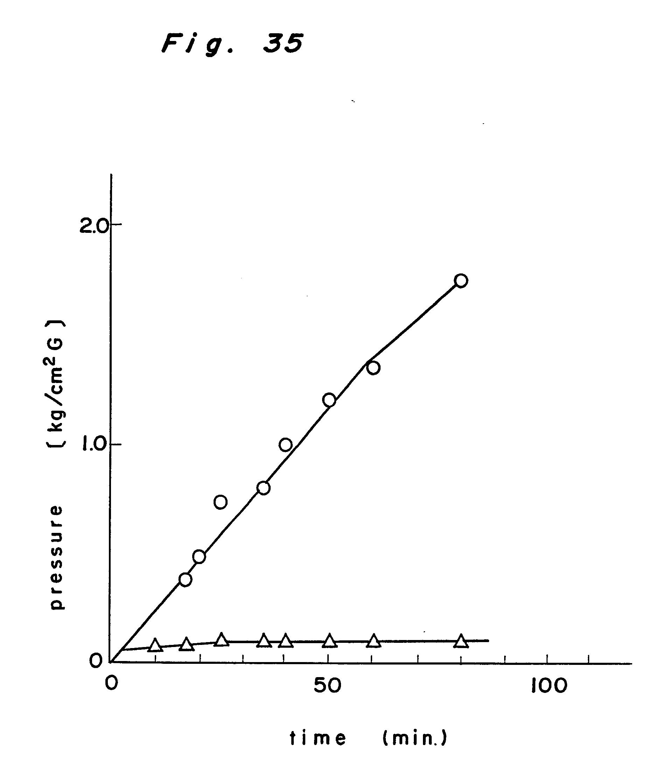

Fig. 35 shows a graph showing pressure rise at the inlet port of the column 77 due to the jam of the insoluble substances in which circular marks show the pressure in case the test solution containing the insoluble substances is fed to the column in one direction continuously and marks $\Delta$ show the pressure when the test solution containing the insoluble substances is fed to the column 77 for five minutes and in subsequent five minutes, the buffer solution is flown in the column 77 in the counter direction and this operation is repeated.

As understood from the graphs shown in Fig. 35, in case the test solution is fed to the column in one direction continuously, there occurs pressure rise at the inlet port, on the other hand, in case the test solution and buffer solution are alternately fed to the column reversing the feeding direction, the pressure rise at the inlet port of the column is little and the insoluble substances can be discharged from the column 77.

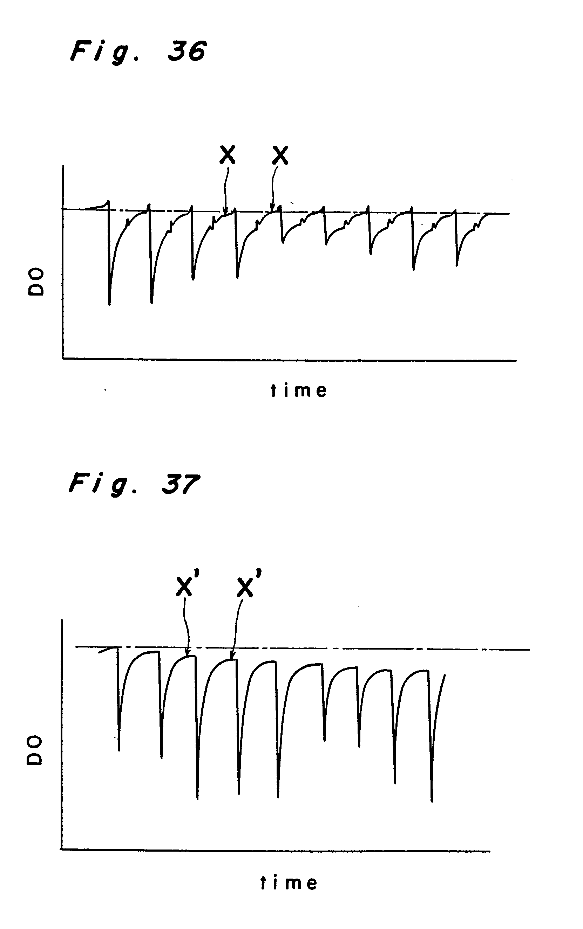

Fig. 36 shows the dissolved oxygen concentration DO measured by the arrangement shown in Figs. 29 and 30 from which it is shown that the base value X of the dissolved oxygen is held substantially constant. In case the flow of the direction of the solution in the column 77 is not reversed, the dissolved oxygen concentration DO is shown in Fig. 37 in which the base value $X'$ of the density is decreased due to the deposition of the insoluble substances. When the base value is decreased, the available BOD measurement range is narrowed and BOD measurement accuracy is deteriorated.

The operation of the device shown in Figs. 29 and 30 under the flow injection method is described hereinafter.

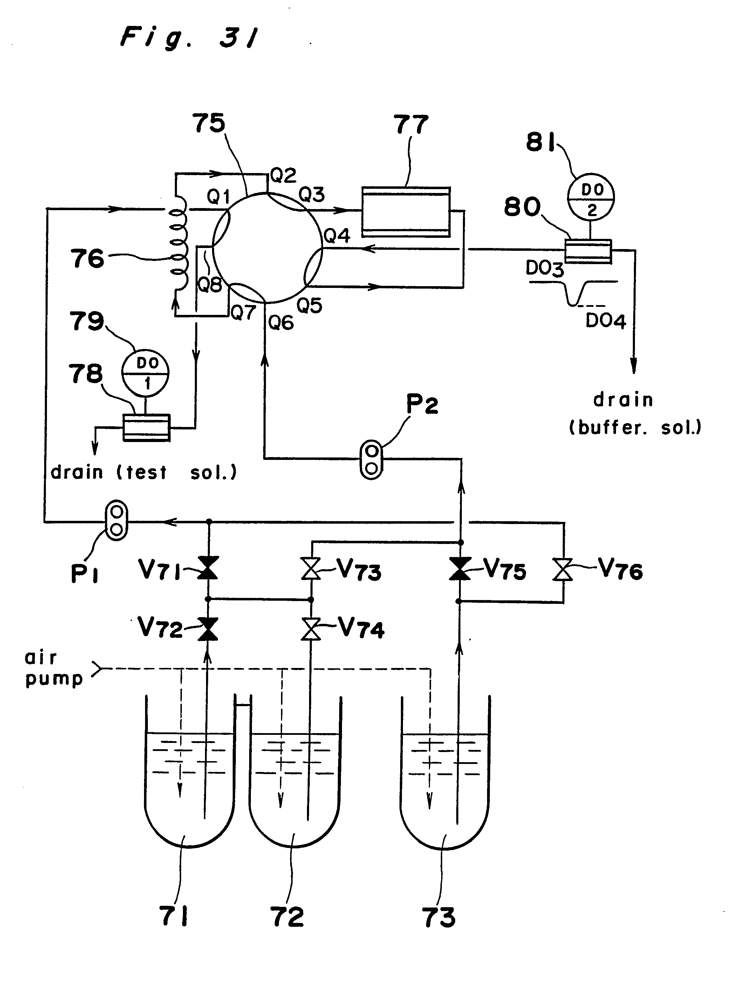

In the load condition, the rotary valve 75 and the valves V71 to V76 are respectively in the same states as shown in Fig. 29 and the test solution 71 is fed to the flow cell 78 through the sample loop 76 and in turn discharged. The buffer solution 73 is passed the column 77 from right to left and fed to the flow cell 80 and the dissolved oxygen concentration $DO_3$ of the buffer 73 is measured by the DO electrode 81. The load condition is continued for 10 minutes for example and the condition is changed to the inject condition as shown in Fig. 31. In the inject condition, the state of the rotary valve 75 is the same as shown in Fig. 30 and the states of the valves V71 to V76 are the same as shown in Fig. 29. In the inject condition, the test solution 71 passes the valves V72 and V71 and the ports Q1 to Q8 of the rotary valve 75 by the pump P1 and in turn fed to the flow cell 78 without passing the sample loop 76, being discharged. On the other hand, the buffer solution 73 passes by the pump P2 such paths as the valve V75, Q6, Q7, sample loop 76, Q2, and Q3 and further passes the column 77 from left to right and reaches the flow cell 80 through the ports Q5 and Q4. In the flow cell 80, the dissolved oxygen concentration of the buffer solution is measured. Since the buffer solution passes the sample loop 76, the test solution 71 stayed in the sample loop 76 in the load condition is pushed and flown to the column 77 by the buffer solution in the inject condition. When the test solution from which oxygen is consumed in the column 77 is passed the flow cell 80, the oxygen concentration of the test solution measured by the DO electrode 81 is decreased temporarily. By expressing the lowest density as $DO_4$, the oxygen decrement $\Delta DO$ of the test solution can be calculated by $\Delta DO = DO_3 - DO_4$.

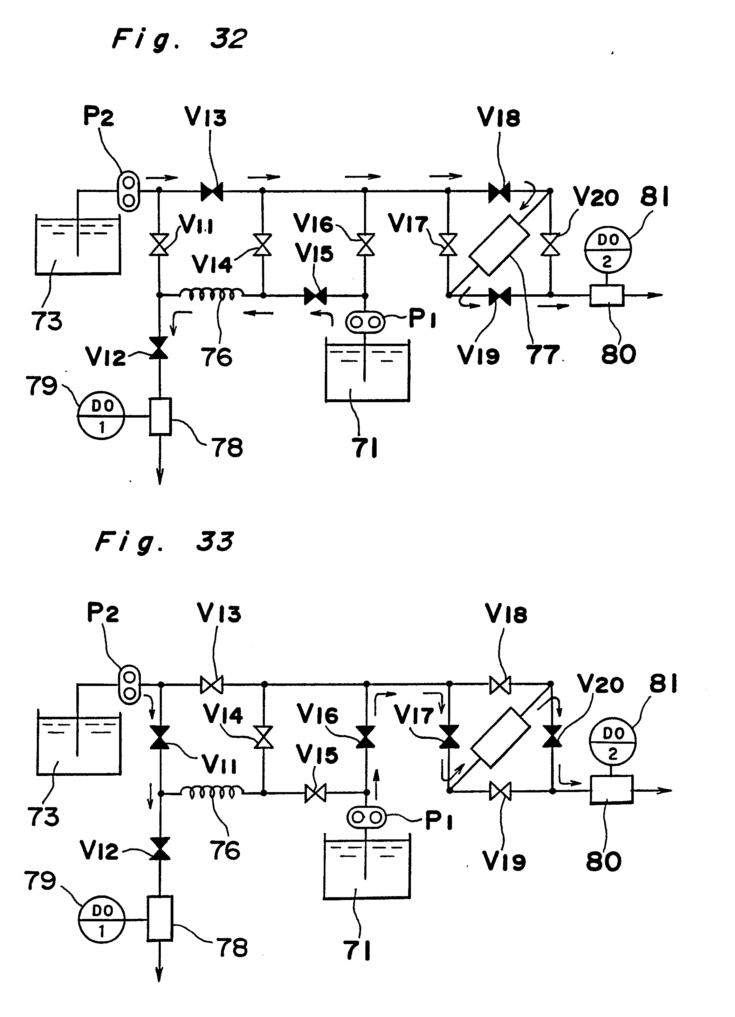

Figs. 32 and 33 show a further modification of the BOD measurement device in which the device shown in Figs. 29 and 30 is arranged by using the two way valves.

Fig. 32 shows the load condition in which the valves V12, V13, V15, V18 and V19 are opened and the buffer solution flows in the column 77 from the common junction E3.

Fig. 33 shows the inject condition in which the valves V11, V12, V16, V17 and V20 are opened and the test solution is fed to the column 77 from the common junction E4. Other operations are similar to those shown in the arrangement in Figs. 29 and 30.

In case the arrangement shown in Figs. 32 and 33 is used for the flow injection method, in the load condition, the states of the valves are the same as shown in Fig. 32. In the inject condition, the valves V11, V14, V17 and V20 are opened and the valves V12, V13, V15, V16, V18 and V19 are closed and the test solution filled in sample loop 76 is pushed and flow out to the common junction E3 by the buffer solution flowing in the column 77 from the common junction E4. Other operations are similar to those in the embodiment shown in the arrangement in Figs. 30 and 31.

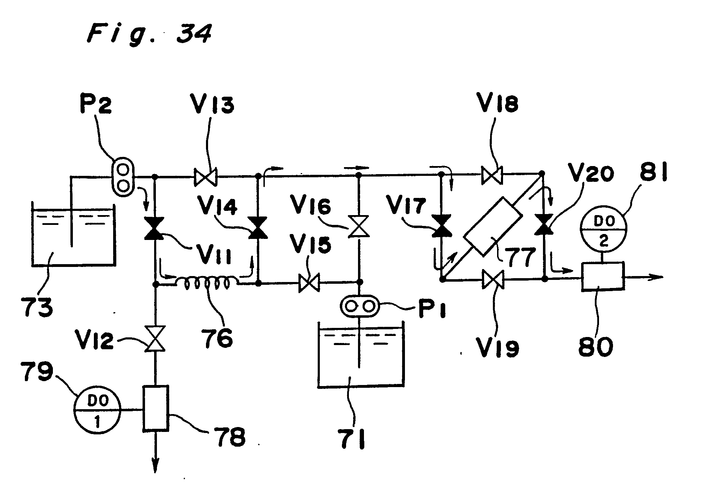

In the arrangements shown in Figs 29 to 34, since the direction of the solution in the column 77 is reversed, the insoluble substance in the column can be discharged out of the column, so that it is possible to prevent jam in the column, therefore the available range of the BOD measurement can be expanded and the accuracy of the measurement can be improved.

## Claims

1. A method of measurement of BOD value of test solution by comparing dissolved oxygen decrement of the test solution with dissolved oxygen decrement of standard solution, the dissolved oxygen decrement of solution being occurred by contacting the solution with immobilized aerobic microorganism, wherein the standard solution is sterilized test solution.

2. The method of measurement of BOD value according to claim 1 or 2, wherein the sterilization of the test solution is carried out by autoclaving method of a pressure sterilization with a temperature higher than $110^{\circ}$ C.

3. The method of measurement of BOD value according to claim 1, wherein the sterilization of the test solution is carried out by ultraviolet sterilization.

4. A method of measurement of BOD value of test solution by comparing dissolved oxygen decrement of the test solution with dissolved oxygen decrement of standard solution, the dissolved oxygen decrement of solution being occurred by contacting the solution with immobilized aerobic microorganism, comprising

a step of calculating an equation between reciprocal value of the dissolved oxygen decrement $1/\Delta DO$ and reciprocal of BOD $1/BOD$ of the standard solution,

a step of measuring the dissolved oxygen decrement of the test solution,

a step of obtaining reciprocal of the dissolved oxygen decrement $1/\Delta DO$ of the test solution, and

a step of obtaining the BOD value of the test solution by using the equation obtained for the standard solution and $1/\Delta DO$ of the test solution.

5. The method of measuring BOD value of test solution according to claim 4, further comprising a step of obtaining constants A and B in the following equation using known BOD value and measured $\Delta DO$ of standard solution.

$$BOD = \frac{B \cdot \Delta DO}{A - \Delta DO}$$

A step of obtaining BOD value of the test solution by placing the measured $\Delta DO$ of the test solution in the above equation.

6. A method of measurement of BOD value of test solution by comparing dissolved oxygen decrement of the test solution with dissolved oxygen decrement of standard solution, the dissolved oxygen decrement of solution being occurred by contacting the solution with immobilized aerobic microorganism, wherein the contact of the solution with the microorganism comprising a step of flowing the solution in a container storing immobilized aerobic microorganism, and

a step of reversing the direction of the flow of the solution in the column at a predetermined time interval.

7. An apparatus for measuring BOD value of test solution by comparing dissolved oxygen decrement of the test solution with dissolved oxygen decrement of standard solution, the dissolved oxygen decrement of solution being occurred by contacting the solution with immobilized aerobic microorganism, which comprises

(a) a first DO sensor for measuring dissolved oxygen concentration in solution,

(b) a column packed with immobilized aerobic organism for contacting the solution therein,

(c) a second DO sensor for measuring dissolved oxygen concentration of the solution fed from said column, and

(d) means for feeding test solution to said column and in turn to said second DO sensor and for feeding buffer solution to said first DO sensor in a inject condition which is such a condition that the test solution is flown through the second DO sensor through said column,

(e) means for feeding the test solution to said first DO sensor and for feeding the buffer sensor to said second DO sensor through. said column in a load condition which is such a condition that the buffer solution is fed to the second DO sensor, and

(f) means for calculating BOD values of the test solution using the measurement values by said first DO sensor and second DO sensor.

8. An apparatus according to claim 7, wherein said apparatus further comprises means for reversing the direction of solution flow in the column at a predetermined time interval.

9. The apparatus according to claim 7 or 8, wherein said apparatus further comprises means for reversing the direction of the solution flow in said column corresponding to the change of the inject condition and the load condition.

Fig. 1

Fig. 2

Fig. 3

Fig. 4

Fig. 5

Fig. 6

*Fig. 7*

Fig. 8

start

S1 — calibration / measurement ?

calibration:

S2 — input BOD

S3 — calib. calculate ΔDO

S4 — finish calibration ?

S5 — calculate equation

measurement:

S11 — mesure, calculate ΔDO

S12 — calculate BOD

S13 — output BOD

Fig. 9

Fig. 10

# Fig. 11

control device — 16

DO sensor — 5

oxygen electrode — 2

5

2

16

10  11  4

12  13

9

19  3

7

to drain

to drain

14

15  6

EP 0 369 490 A2

Fig. 12

# Fig. 13

EP 0 369 490 A2

# Fig. 14

drain

# Fig. 15

*Fig. 16*

*Fig. 17*

Fig. 18

Fig. 19

*Fig. 20*

*Fig. 21*

*Fig. 22*

*Fig. 23*

EP 0 369 490 A2

Fig. 24

Fig. 25

Fig. 26 (Prior Art)

# Fig. 27 (Prior Art)

first　　　　　　　　　second

| load (DO 1b) | inject (DO 1s) | load (DO 1b) | inject (DO 1s) |

DO1

DO [mg/ℓ]

10, 8, 6, 4, 2, 0

Time [min]: 0 10 20 30 40 50 60 70 80

# Fig. 28 (Prior Art)

DO2

DO [mg/ℓ]

(DO 2b) (DO 2s) (DO 2b) (DO 2s)

10, 8, 6, 4, 2, 0

Time (min): 0 10 20 30 40 50 60 70 80

# Fig. 29

Fig. 30

EP 0 369 490 A2

Fig. 31

drain (buffer. sol.)

drain (test sol.)

air pump

Fig. 32

Fig. 33

# Fig. 34

*Fig. 35*

EP 0 369 490 A2

*Fig. 36*

*Fig. 37*